# EUROPEAN PATENT APPLICATION

(11) **EP 2 106 811 A2**
(43) Date of publication of application: **07.10.2009**
(21) Application number: 09004770.5
(22) Date of filing: 31.03.2009
(51) Int. Cl.: A61L 27/14, A61L 27/56

(54) **Scaffold for tissue engineering and production method thereof**

(30) Priority: 31.03.2008 JP 2008091991
(71) Applicant: GC Corporation, Itabashi-ku Tokyo 174-8585 (JP)
(72) Inventor: Yamamoto, Katsushi, Tokyo 174-8585 (JP); Yamanaka, Katsuyuki, Tokyo 174-8585 (JP); Suda, Youko, Tokyo 174-8585 (JP); Sakai, Yuhiro, Tokyo 174-8585 (JP); Kaneko, Tadashi, Tokyo 174-8585 (JP)
(74) Representative: Müller-Boré & Partner Patentanwälte

(57) **Abstract**

To provide a scaffold for tissue engineering which consists of a bioabsorbable polymer material to be absorbed in a biotissue, and holds strength of the whole scaffold while having a porosity proper for culturing cells inside thereof as well, a method for producing the scaffold for tissue engineering includes steps of dissolving a bioabsorbable polymer material with an organic solvent, drying the solution so as to produce a porous bioabsorbable polymer material having a porosity of 50 to 99%, covering the porous bioabsorbable polymer material with a bioabsorbable polymer material having a thickness of 0.01 to 5 mm, pores of 10 to 3000µm diameter, a fracture strength of 0.05 to 0.15MPa, and a volume of 15 to 90% with respect to the whole scaffold.

## Description

The present invention relates to a scaffold for tissue engineering used as a substitute of a biotissue and made of a bioabsorbable polymer material capable of having excellent shape stability and seeding and/or culturing cells in the inside thereof, and a production method thereof.

Recently, a medical operation for regenerating a biotissue lost by an external injury or a surgical operation was carried out by re-organizing the lost biotissue with somatic cells or mesenchymal stem cells and implanting it in a patient. In such a treatment, a scaffold (matrix) until seeded cells rebuild a biotissue is important in order to regenerate the biotissue.

As for a conventional scaffold, for example, Japanese Patent Application Laid-Open No. 10-234844 discloses a sponge-like scaffold for tissue engineering having pore diameters of about 5 to 100µm, which is produced by dissolving a bioabsorbable polymer material made of lactic acid, glycolic acid, and caprolactone and the like with an organic solvent such as dioxane, and freeze-drying the solution. Further, Japanese Translation of PCT Publication No. 2002-541925 discloses a scaffold for cells made of a bioabsorbable polymer material having a porous structure having circular open large pores of about 50 to 500µm and circular open small pores of 20µm or smaller, and the scaffold is produced by taking a water-soluble non-toxic particle-shape material (e.g., sodium chloride powder or the like) having particle diameters of about 50 to 500µm into the solution at the time of producing the aforementioned sponge-like scaffold for tissue engineering, removing the solvent so as to produce a bioabsorbable polymer material containing the particle-shape material, and thereafter removing the particle-shape material by using water or the like. However, these scaffolds made of a bioabsorbable polymer having a porous structure have low strength because of being sponge-like, and thus there is a problem that the scaffolds cannot keep a required shape in a living body.

The present invention is directed to provide a scaffold for tissue engineering made of a bioabsorbable polymer material capable of holding strength of the scaffold as well as having enough spaces for spreading cells to the inside, and a production method thereof.

Present inventors carried out earnest works to solve the aforementioned problems and, as a result, they found out the followings to complete the present invention. A comparatively soft porous bioabsorbable polymer material having a porosity of 50 to 99%, which is proper for culturing cells, is covered with a hard bioabsorbable polymer material having pores of 10 to 3000µm diameter, through which body liquid, culture liquid, and cells can pass, and having a volume of 15 to 90% of the whole scaffold. As a result, a scaffold for tissue engineering can hold strength and have a proper porosity for culturing cells as well.

According to an aspect of the present invention, a scaffold for tissue engineering is structured such that a periphery of a porous bioabsorbable polymer material having a porosity of 50 to 99% is covered with a bioabsorbable polymer material having a thickness of 0.01 to 5 mm, pores of 10 to 3000µm diameter, a fracture strength of 0.05 to 0.15MPa, and a volume of 15 to 90% with respect to the whole scaffold. A method for producing the scaffold for tissue engineering includes steps of dissolving a bioabsorbable polymer material with an organic solvent, drying the solution so as to produce a porous bioabsorbable polymer material having a porosity of 50 to 99%, covering the produced porous bioabsorbable polymer material with a divided capsule made of a bioabsorbable polymer material having a thickness of 0.01 to 5 mm, pores of 10 to 3000µm diameter, and a fracture strength of 0.05 to 0.15MPa, and having a volume of 15 to 90% with respect to the whole scaffold, and welding the divided capsule together.

In the production method of the scaffold for tissue engineering, the divided capsule, which is a bioabsorbable polymer material, is welded together preferably by heating or through an organic solvent for dissolving the bioabsorbable polymer material.

The present invention is a scaffold for tissue engineering consisting of a bioabsorbable polymer material to be absorbed in a biotissue, and holding strength of the whole scaffold as well as having a porosity suitable for culturing cells inside thereof.

According to an aspect of the present invention, a scaffold for tissue engineering is structured such that a periphery of a porous bioabsorbable polymer material having a porosity of 50 to 99% is covered with a bioabsorbable polymer material having a thickness of 0.01 to 5 mm, pores of 10 to 3000µm diameter, a fracture strength of 0.05 to 0.15MPa, and a volume of 15 to 90% with respect to the whole scaffold. A method for producing the scaffold for tissue engineering includes the steps of dissolving a bioabsorbable polymer material with an organic solvent, drying the solution so as to produce a porous bioabsorbable polymer material having a porosity of 50 to 99%, covering the produced porous bioabsorbable polymer material with a divided capsule made of a bioabsorbable polymer material, having a thickness of 0.01 to 5 mm, pores of 10 to 3000µm diameter, and a fracture strength of 0.05 to 0.15MPa, and having a volume of 15 to 90% with respect to the whole scaffold, and welding the divided capsule together.

The bioabsorbable polymer material used in the present invention is not restricted especially if it is safe for a living body and can hold a shape within a certain period of time. For example, at least one kind selected from polyglycolic acid, polylactic acid, a lactic acid / glycolic acid copolymer, poly-ε-caprolactone, a lactic acid / ε-caprolactone copolymer, polyamino acid, polyorthoester, and a copolymer of those, can be used. Among those, polyglycolic acid, polylactic acid, and a lactic acid / glycolic acid copolymer are preferable because of being approved as a polymer nontoxic to human bodies by U.S. Food and Drug Administration (FDA) and in view of past good result. The weight average molecular weight of the bioabsorbable polymer material is preferably 5,000 to 2,000,000, and more preferably 10,000 to 500,000.

The organic solvent for dissolving the bioabsorbable polymer material is selected properly depending on a bioabsorbable polymer material to be used. However, generally, at least one kind selected from chloroform, dichloromethane, carbon tetrachloride, acetone, dioxane, and tetrahydrofuran can be preferably used. During a dissolving process, a thermal treatment or a supersonic treatment can be used together. A concentration of the bioabsorbable polymer is not restricted especially if it can be dispersed uniformly in the organic solvent, but the concentration is preferably 1 to 20% by weight in the organic solvent.

As for a drying method for removing the organic solvent, a natural drying method under a ventilated condition at an ordinary temperature and an ordinary pressure or a freeze-drying method can be used. In a case of the natural drying method under a ventilated condition at an ordinary temperature and an ordinary pressure, since there are a few pores in a dried sheet-like bioabsorbable polymer material, pores of 0.1 to 3 mm diameter can be mechanically given by punching or the like. In a case of the freeze-drying method, small pores having a pore diameter of about 50µm are formed in the sheet-like bioabsorbable polymer. Thus, the freeze-drying method is preferable because a body liquid and a culture liquid can well spread.

In the scaffold for tissue engineering according to the present invention, the porous bioabsorbable polymer material configuring the inside of the scaffold can be produced by a conventionally used production method, but is necessarily produced so as to have porosity of 50 to 99%. The porosity in the present invention is a numerical value indicated with (1-W₁/W₂)×100 where a weight of a material having pores is W₁ and a weight of a material having no pore is W₂ in a case of using the same materials having the same volume.

As for the porous bioabsorbable polymer material, if the porosity is less than 50%, the efficiency of culturing cells is insufficient because spaces are few, so it is not preferable. If the porosity is more than 99%, an amount of the bioabsorbable polymer is small and thus the function as a scaffold of cells decreases. As a result, since the efficiency of culturing cells is insufficient, it is not preferable.

In the scaffold for tissue engineering according to the present invention, as the porous bioabsorbable polymer material configuring the inside of the scaffold, the followings can be used. A sponge-like porous bioabsorbable polymer material having a pore diameter of 5 to 100µm is produced by dissolving a bioabsorbable polymer material with an organic solvent, freeze-drying the solution. Another porous bioabsorbable polymer material having a circular open large diameter pore of about 50 to about 500µm and a circular open small diameter pore of about 20µm or less is produced by taking a water-soluble non-toxic particle-shape material (e.g., sodium chloride powder or the like) having a particle diameter of about 50 to 500µm into the solution, removing the solvent so as to produce a bioabsorbable polymer material containing the particle-shape material, and thereafter dissolving and removing the particle-shape material by using water or the like. Another porous bioabsorbable polymer material is produced by almost uniformly mixing a solution of a bioabsorbable polymer material being dissolved in an organic solvent and a particle-shape material having a diameter of 100 to 2000µm, which is not dissolved with the organic solvent but dissolved with a liquid which does not dissolve the bioabsorbable polymer material, freezing and then drying the mixture, removing thereby the organic solvent so as to produce a porous bioabsorbable polymer material containing the particle-shape material and having a small pore structure having a pore diameter of 5 to 50µm, pulverizing the produced porous bioabsorbable polymer material, dissolving and removing the particle-shape material with the liquid which does not dissolve the bioabsorbable polymer, sieving the remainder to obtain a bioabsorbable granular porous material having particle diameters of 100 to 3000µm, taking the bioabsorbable granular porous material into a container having a predetermined shape, and pressurizing and heating it.

In the scaffold for tissue engineering according to the present invention, it is necessary that the bioabsorbable polymer material configuring an outline part has a thickness of 0.01 to 5 mm, a pore diameter of 10 to 3000µm, a fracture strength of 0.05 to 0.15MPa, and a volume of 15 to 90% of the whole scaffold.

If the pore diameter is less than 10µm, since the size of the pore is small, the efficiency for culturing cells is insufficient. So, it is not preferable. If the pore diameter is more than 3000µm, the bioabsorbable polymer material lessens its function as a scaffold of cells. As a result, the efficiency of culturing cells is insufficient, and it is not preferable.

If the fracture strength is less than 0.05MPa, the bioabsorbable polymer material cannot hold a shape needed in a living body regardless of a kind of the shape. On the other hand, it is hard to make the bioabsorbable polymer having pores and the fracture strength of more than 0.15MPa. In addition, the fracture strength in the present invention means the compressive fracture strength when a cylindrical test body having a diameter of 10 mm and a height of 2 mm is compressed at a crosshead speed of Imm/min.

If the volume is less than 15% of the whole scaffold, the strength required for the scaffold for tissue engineering cannot be kept, so it is not preferable. If the volume is more than 90%, the porous bioabsorbable polymer material configuring the inside for culturing cells decreases, and thus the efficiency for culturing cells is insufficient. So, it is not preferable.

If the thickness is less than 0.01 mm or more than 5 mm, the strength of the produced scaffold for tissue engineering becomes insufficient, or a space for proliferating cells becomes useless.

It is necessary that the bioabsorbable polymer material configuring the outline part can cover the periphery of the porous bioabsorbable polymer material configuring the inside of the support for tissue engineering. As for this bioabsorbable polymer material configuring the outline part, the followings can be used. A sheet-shape bioabsorbable polymer material having a thickness of 0.01 to 5 mm is produced by dissolving a bioabsorbable polymer material with an organic solvent, thinly extending the organic solvent in which the bioabsorbable polymer material is dissolved, drying the solution so as to remove the organic solvent, and pressing the remaining bioabsorbable polymer material if necessary. Further, another bioabsorbable polymer material consisting of a divided capsule capable of housing a porous bioabsorbable polymer material is produced by cutting the sheet-shape bioabsorbable polymer material produced by the aforementioned method into a strip shape, collectively taking the material into a mold, and pressurizing, heating and molding the material.

As for another example, the following bioabsorbable polymer material can be used. A bioabsorbable polymer material consisting of divided capsules capable of housing a porous bioabsorbable polymer material is produced by almost uniformly mixing a solution of a bioabsorbable polymer material being dissolved in an organic solvent and a particle-shape material having a diameter of 100 to 2000µm, which is not dissolved with the organic solvent but dissolved with a liquid which does not dissolve the bioabsorbable polymer material, thereafter drying the mixture and removing the organic solvent so as to produce a polymer material containing a particle-shape material and having a small pore structure of a pore diameter of 5 to 50µm, pulverizing the produced polymer material, dissolving and removing the particle-shape material with the liquid which does not dissolve the bioabsorbable polymer, sieving the remainder to obtain a bioabsorbable granular porous material having particle diameters of 100 to 3000µm, taking the bioabsorbable granular porous material into a mold, and pressurizing and heating the material so as to have a needed fracture strength.

In the scaffold for tissue engineering according to the present invention, although the heating condition for producing the porous bioabsorbable polymer material configuring the inside of the scaffold and the bioabsorbable polymer material configuring the outline part is changed depending on the material, shape, and size of the bioabsorbable polymer material, the heating condition can be within a range of 60 to 200°C. If the heating condition is less than 60°C, the bonding between the bioabsorbable polymer material decreases, and thus it is hard to keep the shape for configuring the outline part. On the other hand, if the heating condition is more than 200°C, the bioabsorbable polymer may be denatured.

In a case that the outline part of the scaffold for tissue engineering consists of the divided capsules made of the bioabsorbable polymer material, the divided capsules are preferably welded by any one of a method for welding the divided capsules by heating, and a method for welding them through an organic solvent capable of dissolving the bioabsorbable polymer material. The organic solvent in which the bioabsorbable polymer material is previously dissolved can be used.

### [Example 1]

Production of a devided capsule made of a bioabsorbable polymer material for the outline of a scaffold:
A polymer material was produced by taking a lactic acid / glycolic acid copolymer (lactic acid : glycolic acid = 75:25, a weight average molecular weight of about 250,000) into dioxane so as to have a concentration of 12% by weight, stirring and dissolving the mixture by a stirring machine, almost uniformly mixing sodium chloride powder (having a particle diameter of 300 to 700µm) with the dioxane solution, in which the lactic acid / glycolic acid copolymer was dissolved, so as to have a concentration of about 1.18 g/cm³, taking the mixture into a mold, freezing the mixture at -30°C by a freezer (a product name: MDf-0281AT, produced by SANYO Electric Co., Ltd.), and drying the frozen mixture under reduced pressure for 48 hours by a vacuum dryer (a product name: DP43, produced by Yamato Scientific Co., Ltd.) so as to remove dioxane. The produced polymer material contained sodium chloride powder almost uniformly. Then, a bioabsorbable granular porous material was produced by cutting this polymer material to be small pieces, pulverizing the small pieces for 50 minutes by a pot mill for planetary rotation, taking the pulverized polymer material into a flask, adding distilled water to the flask, stirring the mixture so as to remove sodium chloride, transferring the polymer material to a laboratory dish, drying it for 48 hours by the vacuum dryer, and sieving the polymer material. The produced bioabsorbable granular porous material had a particle diameter of 300 to 700µm and an average pore diameter of about 5µm. Then, a bioabsorbable polymer material was produced by sealing 0.01g of the bioabsorbable granular porous material in a titanium mold having an inner diameter of 10 mm and a height of 20 mm and having a sealed lower bottom, and heating the material for 10 minutes at 80°C while keeping a volume in a state of being pressed at by 1500 g/cm² from an upper part by a titanium rod having an outer diameter of 10 mm and a projection having a diameter of 8 mm and a height of 1 mm at a center part. The produced two divided capsules made of bioabsorbable polymer material having an outer diameter of 10 mm, a height of 2 mm, and a recessed part having a diameter of 8 mm and a depth of 1 mm at a center part.

Further, a cylindrical test body having a diameter of 10 mm and a height of 2 mm was produced by a similar method to the aforementioned method and compressed at a crosshead speed of 1 mm/min. The fracture strength of the bioabsorbable polymer material for an outline was 0.1 MPa.
Production of a porous bioabsorbable polymer material for the inside of the scaffold:
A polymer material was produced by taking a lactic acid / glycolic acid copolymer (lactic acid : glycolic acid = 75:25, a weight average molecular weight of about 250,000) into dioxane so as to have a concentration of 12% by weight, stirring and dissolving the mixture by the stirring machine, almost uniformly mixing sodium chloride powder (having a particle diameter of 300 to 700µm) with the dioxane solution in which the lactic acid / glycolic acid copolymer was dissolved so as to have a concentration of 1.18 g/cm³, taking the mixture into a glass container having a sealed lower bottom, an inner diameter of 8 mm and a height of 10 mm so as to have a height of about 3 mm, freezing the mixture at -30°C by a freezer (a product name: MDf-0281AT, produced by SANYO Electric Co., Ltd.), drying the frozen mixture under reduced pressure for 48 hours by a vacuum dryer (a product name: DP43, produced by Yamato Scientific Co., Ltd.), and removing dioxane so as to obtain a polymer material containing sodium chloride almost uniformly. A porous bioabsorbable polymer material was produced by adding distilled water to the obtained polymer material so as to remove sodium chloride, and drying the polymer material for 48 hours by the vacuum dryer. The produced porous bioabsorbable polymer material was sponge-like and had a cylindrical shape having a diameter of 8 mm and a height of 1.8 mm, an average pore diameter of 300 to 700µm and a small pore structure on a wall face, in which an average pore diameter was about 5µm. When the porosity of this porous bioabsorbable polymer material was measured, it was about 82%.
Production of a scaffold for tissue engineering:
A scaffold for tissue engineering was produced by housing the sponge-like porous bioabsorbable polymer material mentioned above into the capsule consisting of the two divided capsules mentioned above which is the bioabsorbable polymer material, and heating the capsule for 1 minute at 80°C in a state of contacting end faces of the divided capsules so as to weld the end faces of the divided capsules. The produced scaffold for tissue engineering had such a structure that the periphery of the internal sponge-like porous bioabsorbable polymer material for culturing cells was covered with the hard bioabsorbable polymer material, and had an outer diameter of 10 mm and a height of 4 mm.

### [Example 2] Production of a devided capsule made of a bioabsorbable polymer material for the outline of a scaffold:

A sheet-shape bioabsorbable polymer material was acquired by taking a lactic acid / ε-caprolactone copolymer (a weight average molecular weight of about 400,000) in dichloromethane so as to have a concentration of 10% by weight, stirring and dissolving the mixture by the stirring machine, flowing the solution onto a glass plate to meet a condition of 0.1 g/cm², freezing the mixture at -30°C by a freezer (a product name: MDf-0281AT, produced by SANYO Electric Co., Ltd.), drying the frozen mixture under reduced pressure for 48 hours by a vacuum dryer (a product name: DP43, produced by Yamato Scientific Co. , Ltd.), removing dichloromethane so as to acquire a bioabsorbable polymer material having a thickness of 1 mm, and thereafter pressing the acquired bioabsorbable polymer material so as to produce the bioabsorbable polymer material having a thickness of 0.25 mm. Then, a bioabsorbable polymer material was produced by cutting this material to be small pieces having a length of about 10 mm and a width of about 5 mm, charging eight cut strip-shape bioabsorbable polymer materials into a titanium mold having a sealed lower bottom, an inner diameter of 10 mm and a height of 20 mm so as to be entangled irregularly, and heating the materials for 10 minutes at 120°C while keeping a volume in a state of being pressed at 1500 g/cm² from an upper part of the mold by a titanium rod having an outer diameter of 10 mm and a projection having a diameter of 8 mm and a height of 1 mm at a center part. The produced bioabsorbable polymer material consisted of two divided capsules having an outer diameter of 10 mm, a height of 2 mm, and a recessed part having a diameter of 8 mm and a depth of 1 mm at a center part.

Further, a cylindrical test body having a diameter of 10 mm and a height of 2 mm was produced by a similar method to the aforementioned method and compressed at a crosshead speed of 1 mm/min. The fracture strength of the bioabsorbable polymer material for an outline was 0.08MPa.
Production of a porous bioabsorbable polymer material for the inside:
A sponge-like porous bioabsorbable polymer material produced by a similar method to that of Example 1 was used.
Production of a scaffold for tissue engineering:
A scaffold for tissue engineering was produced by housing the sponge-like porous bioabsorbable polymer material into the capsule consisting of the two divided capsules mentioned above which was the bioabsorbable polymer material, coating the following solution to the end faces of the divided capsules, and welding the end faces of the divided capsules. The solution was made by taking a lactic acid / ε-caprolactone copolymer (a weight average molecular weight of about 400,000) in dichloromethane so as to have a concentration of 10% by weight and stirring and dissolving the mixture by a stirring machine. The produced scaffold for tissue engineering had such a structure that the periphery of the internal sponge-like porous bioabsorbable polymer material for culturing cells was covered with the hard bioabsorbable polymer material. The outer diameter of the scaffold was 10 mm and the height was 4 mm.

## Claims

1. A scaffold for tissue engineering, wherein a periphery of a porous bioabsorbable polymer material having porosity of 50 to 99% is covered with a bioabsorbable polymer material having a thickness of 0.01 to 5 mm, pores of 10 to 3000µm diameter, a fracture strength of 0.05 to 0.15MPa, and a volume of 15 to 90% with respect to the whole scaffold.

2. A production method of a scaffold for tissue engineering, comprising steps of:
dissolving a bioabsorbable polymer material with an organic solvent;
drying the solution so as to produce a porous bioabsorbable polymer material having a porosity of 50 to 99%;
covering the porous bioabsorbable polymer material with a divided capsule made of a bioabsorbable polymer material having a thickness of 0.01 to 5 mm, pores of 10 to 3000µm diameter, and a fracture strength of 0.05 to 0.15MPa, and having a volume of 15 to 90% with respect to the whole scaffold; and
welding the divided capsule together.

3. The production method of a scaffold for tissue engineering as claimed in claim 2, wherein the divided capsule is welded by heating.

4. The production method of a scaffold for tissue engineering as claimed in claim 2, wherein the divided capsule is welded through an organic solvent for dissolving a bioabsorbable polymer material.
